# EUROPÄISCHE PATENTANMELDUNG

(11) **EP 2 305 112 A1**
(43) Veröffentlichungstag der Anmeldung: **06.04.2011**
(21) Anmeldenummer: 09171974.0
(22) Anmeldetag: 01.10.2009
(51) Int. Cl.: A61B 5/053

(54) **Bioimpedanzmessvorrichtung**

(71) Anmelder: seca ag, 4153 Reinach BL 1 (CH)
(72) Erfinder: Willers, Frank, 23554, Lübeck (DE)
(74) Vertreter: Ahme, Johannes

(57) **Zusammenfassung**

Die Erfindung betrifft eine Bioimpedanzmessvorrichtung zur Bestimmung von Zusammensetzungsdaten des menschlichen Körpers mit mehreren Elektroden (15, 16, 17, 18), Messschaltungen (2, 10) auf einer Hauptplatine (1) einschließlich von Spannungsmessschaltungen (10) und einer Steuer- und Auswerteeinheit (2), die dazu vorbereitet ist, nach Maßgabe von vorbestimmten Messprogrammen jeweils über eine für das jeweilige Messprogramm spezifische Elektrode (15) einen Wechselstrom aus einer steuerbaren Wechselstromquelle (4) in den Körper einzuprägen und über eine andere Elektrode (18) Wechselstrom abzuleiten und mit zwei weiteren Elektroden (16, 17) mit den Spannungsmessschaltungen (10) die resultierenden Spannungen festzustellen und daraus die Impedanz von Körpersegmenten zu bestimmen. Erdfindungsgemäß ist jede stromeinprägende Elektrode (15) mit einer von der Hauptplatine (1) separaten, in der Nähe der stromeinprägenden Elektrode (15) angeordneten, von der Steuer- und Auswerteeinheit (2) ferngesteuerten Wechselstromquelle (4) versehen.

## Beschreibung

Die vorliegende Erfindung betrifft eine Bioimpedanzmessvorrichtung zur Bestimmung von Zusammensetzungsdaten des menschlichen Körpers.

Die Leitfähigkeit des menschlichen Körpers wird stark durch den Wasseranteil beeinflusst. Da die fettfreien Anteile des Körpers wie Muskeln und die Körperflüssigkeiten einen Großteil des körpereigenen Wassers beinhalten, während Fettgewebe einen nur relativ geringen Wasseranteil besitzt, kann durch die Bestimmung der Leitfähigkeit des Körpers oder eines Köpersegments (oder umgekehrt des Widerstands oder der Impedanz des Körpers oder des Körpersegments) ein Rückschluss auf den relativen Anteil von Fett gezogen werden, zumindest wenn weitere Daten wie die Größe und das Gewicht der Person berücksichtigt werden.

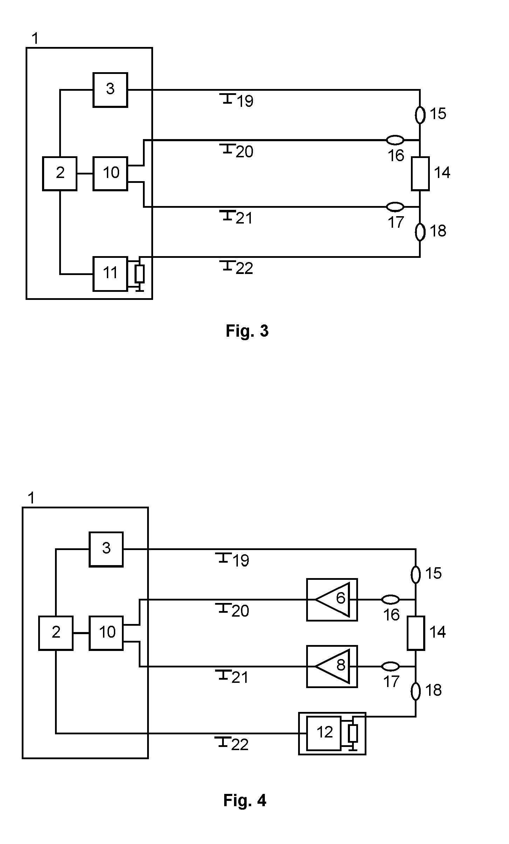

Typische Bioimpedanzvorrichtungen weisen acht Elektroden auf, nämlich vier Fußelektroden, jeweils zwei zum Kontaktieren eines Fußes, und vier Handelektroden, jeweils zwei zur Kontaktierung an einer Hand der Person. Für jedes der Gliedmaßen ist eine stromeinprägende Elektrode vorgesehen. Dann wird ein Wechselstrom über eine Elektrode eingeprägt und über eine andere, an einem anderen der Gliedmaßen befindliche Elektrode abgeleitet und mit weiteren zwei Elektroden, die ebenfalls an unterschiedlichen Gliedmaßen anliegen, die Spannung gemessen. Durch Übergang zu anderen Paaren von stromeinprägenden/ableitenden Elektroden und spannungserfassenden Elektroden können sukzessive verschiedene Körpersegemente untersucht werden. Ferner kann bei Stromeinspeisung in eine Hand und einen Fuß und Spannungsmessung an derselben Hand und an demselben Fuß eine ganze Körperseite gemessen werden. Die meisten Messschaltungen wie auch die Steuer- und Auswerteeinheit sind auf einer Hauptplatine angeordnet, die über Kabel mit den entfernt an den Gliedmaßen angeordneten Elektroden verbunden sind. Insbesondere befinden sich auch die Wechselstromquellen, die einen Wechselstrom mit von der Steuer- und Auswerteeinheit vorgegebener Amplitude erzeugen, um diesen über Elektroden einzuprägen, auf der Hauptplatine. Ein solcher Schaltungsaufbau ist schematisch in Figur 3 dargestellt. Hier befindet sich auf der Hauptplatine 1 die Steuer- und Auswerteeinheit 2, die mit einer Spannungsmessschaltung 10 verbunden ist, die wiederum mit Elektroden 16 und 17 verbunden sind. Die Steuer- und Auswerteeinheit 2 empfängt ebenfalls das Ausgabesignal einer Strommessschaltung 11. Ferner liefert die Steuer- und Auswerteeinheit 2 ein Steuersignal, mit dem die Amplitude der Wechselstromquelle 3 vorgegeben wird. Aus der Wechselstromquelle 3 wird der Wechselstrom über ein Kabel 19 und weiter über die Elektrode 15 in den Körper eingeprägt. Bei dieser Anordnung wird der Strom über die Leitungen 19 und 22 eingeprägt und die Spannung über die Elektroden 16 und 17 gemessen. Für eine genaue Bestimmung der Impedanz 14 muss der in den Körper eingeprägte Strom möglichst genau bekannt sein. In dieser Hinsicht ist der in Figur 3 dargestellte Aufbau nachteilig, da der in der Wechselstromquelle 3 erzeugte Wechselstrom über eine relativ lange Leitung zu seinem Einprägungsort an der Elektrode am Körper geleitet werden muss. Parasitäre Kapazitäten der Leitung sorgen hier insbesondere bei höherfrequenten Wechselströmen für Verluste, so dass der tatsächlich eingeprägte Wechselstrom nicht genau bekannt ist. Infolgedessen ist auch die Impedanz nicht mit hoher Genauigkeit bestimmbar.

Eine Vorrichtung zur Bioimpedanzanalyse nach dem Oberbegriff von Anspruch 1 ist in WO 97/01303 beschrieben. Der schematische Schaltungsaufbau ist in Figur 4 dargestellt, der in weiten Teilen dem zuvor zu Figur 3 beschriebenem Aufbau entspricht, bei dem jedoch die Vorverstärker 6 und 8 separat von der Hauptplatine 1 näher an die spannungsmessenden Elektroden angeordnet sind und ihre Ausgabesignale an die Spannungsmessschaltung 10 weitergeben. Ferner ist separat von der Hauptplatine eine Strommessschaltung 12 angeordnet, mit der der durch die Elektrode 18 abfließende Strom bestimmt und an die Steuer- und Auswerteeinheit 2 gemeldet wird. Auch bei dieser Anordnung wird der einzubringende Wechselstrom durch eine Wechselstromquelle 3 auf der Hauptplatine erzeugt und erleidet bei der Weiterleitung zur einprägenden Elektrode 15 nicht genau vorhersagbare Verluste durch parasitäre Kapazitäten. Bei dieser Anordnung wird der tatsächlich fließende Strom jedoch mit der Strommessschaltung 12 gemessen, so dass er mit der über die Elektroden 16 und 17 gemessenen Spannung zur Bestimmung der Impedanz herangezogen werden kann. Allerdings wird bei diesem Aufbau die zusätzliche Strommessschaltung 12 benötigt.

Es ist Aufgabe der vorliegenden Erfindung, eine Bioimpedanzmessvorrichtung mit einfachem Aufbau anzugeben, mit der die Impedanz mit hoher Genauigkeit bestimmbar ist. Insbesondere soll dies durch präzise Einstellbarkeit des eingeprägten Stroms erreicht werden.

Zur Lösung dieser Aufgabe dienen die Merkmale des Patentanspruchs 1. Vorteilhafte Ausführungsformen der Erfindung sind in den Unteransprüchen aufgeführt.

Erfindungsgemäß ist vorgesehen, dass jede stromeinprägende Elektrode mit einer von der Hauptplatine separaten, in der Nähe der stromeinprägenden Elektrode angeordneten und von der Steuer- und Auswerteeinheit ferngesteuerten Wechselstromquelle versehen ist. Dadurch kann direkt am Ort der stromeinprägenden Elektrode ein Wechselstrom mit vorgegebener Amplitude erzeugt und in die Elektrode eingespeist werden. Auf diese Weise ist die Amplitude des eingeprägten Wechselstroms gut bekannt und wird nicht durch parasitäre Kapazitäten auf dem Weg zur Elektrode verfälscht. Von der Steuer- und Auswerteeinheit auf der Hauptplatine müssen zu jeder Stromquelle lediglich Steuersignale übertragen werden, die die Amplitude des zu erzeugenden Wechselstroms vorgeben. Diese Steuersignale können allerdings weit weniger störanfällig von der Steuer- und Auswerteeinheit zu den entfernten Elektroden übertragen werden, so dass dadurch keine Unsicherheit in der tatsächlich erzeugten Amplitude des Wechselstroms auftritt. Mit der gut bekannten Amplitude des eingeprägten Wechselstroms an der Elektrode kann nun einfach die Spannung über zwei weitere Elektroden gemessen werden und daraus schließlich die Impedanz bestimmt werden, ohne dass eine weitere separate Strommessung des tatsächlich eingeprägten Wechselstroms oder abfließenden Wechselstroms notwendig wäre. Die Strommessschaltung kann daher entfallen. Auf diese Weise kann ein schaltungstechnisch einfacherer Aufbau realisiert werden, mit dem gleichwohl die Impedanz mit guter Genauigkeit messbar ist.

In einer vorteilhaften Ausführungsform sind die Vorverstärker für die spannungsmessenden Elektroden ebenfalls in der Nähe der jeweiligen Elektrode angeordnet, um so die durch den Leitungsweg bedingte Kapazität am Vorverstärkereingang zu minimieren.

In einer vorteilhaften Ausführungsform erfolgt die Fernsteuerung der Wechselstromquelle durch die Steuer- und Auswerteeinheit mittels eines differenziellen Signals, das über eine Twisted-Pair-Leitung zu der Wechselstromquelle geleitet wird. Alternativ kann die Ansteuerung der Wechselstromquelle mit einem Signal erfolgen, das über eine abgeschirmte Leitung zu der Wechselstromquelle geleitet wird.

In bevorzugten Ausführungsformen können für die Übertragung der Spannungsmesssignale aus den nahe der Spannungsmesselektroden angeordneten Vorverstärkern differenziell zu übertragende Signale über Twisted-Pair-Leitungen zu der Steuer- und Auswerteeinheit übertragen werden oder absolute Signale über abgeschirmte Leitungen zu der Steuer- und Auswerteeinheit geleitet werden. Ferner kann in einer bevorzugten Ausführungsform jede stromableitende Elektrode mit der Steuer- und Auswerteeinheit über eine abgeschirmte Leitung verbunden sein.

Die Erfindung wird im Folgenden anhand von Ausführungsbeispielen in den Zeichnungen erläutert, in denen:
Figur 1 einen schematischen Schaltplan für eine Bioimpedanzmessvorrichtung zeigt,
Figur 2 einen schematischen Schaltplan für eine alternative Ausführungsform für eine Bioimpedanzmessvorrichtung zeigt,
Figur 3 einen schematischen Schaltplan für eine Bioimpedanzmessvorrichtung zum Stand der Technik zeigt, und
Figur 4 einen schematischen Schaltplan für eine Bioimpedanzmessvorrichtung zum Stand der Technik zeigt.

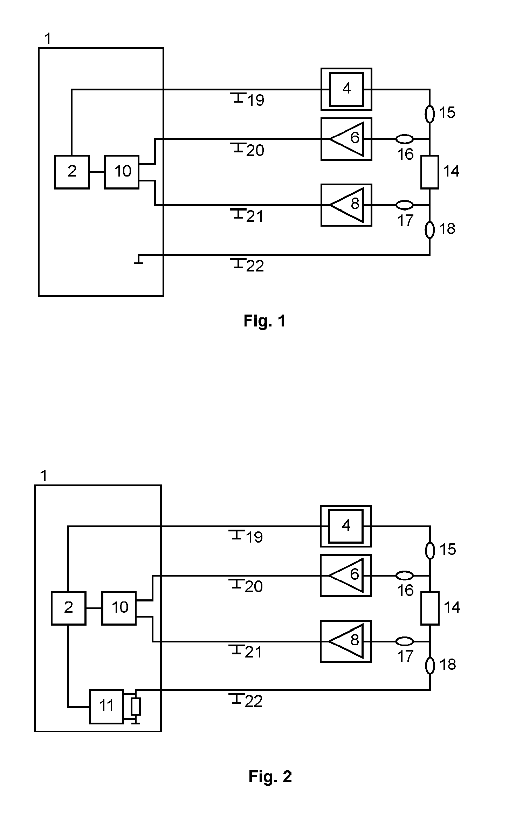

In den in Figuren 1 und 2 der vorliegenden Erfindung gezeigten Ausführungsformen befindet sich die Wechselstromquelle 4, mit der ein Wechselstrom über die Elektroden 15 in den Körper eingeprägt wird, als separates Bauelement entfernt von der Hauptplatine 1 und in der Nähe der einprägenden Elektrode 15. Auf diese Weise kann es nicht zu Verfälschungen des Signals auf dem Weg von der Hauptplatine zu den entfernt an den Gliedmaßen liegenden Elektroden kommen, da der Wechselstrom in unmittelbarer Nähe der Elektrode von der Wechselstromquelle 4 erzeugt wird, die von der Steuer- und Auswerteeinheit 2 ferngesteuert wird. Der von der Wechselstromquelle 4 erzeugte Wechselstrom mit bekannter, vorgegebener Amplitude kann auf dem Weg zur Elektrode 15 daher nicht wesentlich verfälscht werden und ist daher für die Steuer- und Auswerteeinheit 2 gut bekannt. Dann kann mit Hilfe der Spannungsmessung über die Elektroden 16 und 17, deren Signale über die Vorverstärker 6 und 8 zu der Spannungsmessschaltung 10 geleitet werden, die dem eingeprägten Wechselstrom korrespondierende Spannung bestimmt und daraus die Impedanz abgeleitet werden. Eine separate Strommessung ist dann im Prinzip nicht mehr erforderlich, so dass eine Strommessschaltung bei der in Figur 2 dargestellten Ausführungsform ganz entfallen ist.

Bei der in Figur 1 dargestellten Ausführungsform ist zur Ergänzung noch eine Strommessschaltung 11 auf der Hauptplatine zur Kontrolle vorgesehen.

Bei Verwendung einer relativ guten Wechselstromquelle kann allerdings auf das Vermessen des Stromes verzichtet werden, da der Wechselstrom bei präziser Ansteuerung hinreichend genau bekannt ist. Dadurch kann der gesamte Schaltungsteil zur Strommessung eingespart und damit das Bioimpedanzmessgerät vereinfacht werden.

Ferner ist die Amplitude des eingeprägten Wechselstroms in aufeinanderfolgenden Messungen besser reproduzierbar, da die Amplitude des jeweils eingespeisten Wechselstroms nicht durch veränderte Kabelführungen in aufeinanderfolgenden Messungen verändert werden kann.

## Patentansprüche

1. Bioimpedanzmessvorrichtung zur Bestimmung von Zusammensetzungsdaten des menschlichen Körpers mit mehreren Elektroden (15, 16, 17, 18), Messschaltungen (2, 10) auf einer Hauptplatine (1) einschließlich von Spannungsmessschaltungen (10) und einer Steuer- und Auswerteeinheit (2), die dazu vorbereitet ist, nach Maßgabe von vorbestimmten Messprogrammen jeweils über eine für das jeweilige Messprogramm spezifische Elektrode (15) einen Wechselstrom aus einer steuerbaren Wechselstromquelle (4) in den Körper einzuprägen und über eine andere Elektrode (18) Wechselstrom abzuleiten und mit zwei weiteren Elektroden (16, 17) mit den Spannungsmessschaltungen (10) die resultierenden Spannungen festzustellen und daraus die Impedanz von Körpersegmenten zu bestimmen, **dadurch gekennzeichnet, dass** jede stromeinprägende Elektrode (15) mit einer von der Hauptplatine (1) separaten, in der Nähe der stromeinprägenden Elektrode (15) angeordneten, von der Steuer- und Auswerteeinheit (2) ferngesteuerten Wechselstromquelle (4) versehen ist.

2. Bioimpedanzmessvorrichtung nach Anspruch 1, **dadurch gekennzeichnet, dass** in der Nähe der zur Spannungsmessung verwendeten Elektroden (16, 17) jeweils ein von der Hauptplatine (1) separater Vorverstärker (6, 8) angeordnet ist.

3. Bioimpedanzmessvorrichtung nach Anspruch 1 oder 2, die zusätzlich eine Schaltung (11) zur Messung des eingeprägten Wechselstromes aufweist.

4. Bioimpedanzmessvorrichtung nach einem der Ansprüche 1 bis 3, **dadurch gekennzeichnet, dass** die Elektroden jeweils paarweise einem der vier Gliedmaßen zugeordnet sind.

5. Bioimpedanzmessvorrichtung nach einem der Ansprüche 1 bis 4, **dadurch gekennzeichnet, dass** die Steuerung der Wechselstromquelle (4) durch die Steuer- und Auswerteeinheit (2) mit einem differenziellen Signal, das über eine Twisted-Pair-Leitung zu der Wechselstromquelle geleitet wird, erfolgt.

6. Bioimpedanzmessvorrichtung nach einem der Ansprüche 1 bis 4, **dadurch gekennzeichnet, dass** die Steuerung der Wechselstromquelle (4) durch die Steuer- und Auswerteeinheit (2) mit einem Signal, das über eine abgeschirmte Leitung zu der Wechselstromquelle geleitet wird, erfolgt.

7. Bioimpedanzmessvorrichtung nach Anspruch 2, **dadurch gekennzeichnet, dass** die Spannungsmesssignale aus den elektrodennahen Vorverstärkern (6, 8) zur Hauptplatine (1) als differenzielle Signale über Twisted-Pair-Leitungen zu der Steuer- und Auswerteeinheit (2) übertragen werden.

8. Bioimpedanzmessvorrichtung nach Anspruch 2, **dadurch gekennzeichnet, dass** die Spannungsmesssignale aus den elektrodennahen Vorverstärkern (6, 8) zur Hauptplatine (1) über abgeschirmte Leitungen zu der Steuer- und Auswerteeinheit (2) übertragen werden.

9. Bioimpedanzmessvorrichtung nach einem der Ansprüche 1 bis 8, **dadurch gekennzeichnet, dass** jede Wechselstrom vom Körper ableitende Elektrode mit der Hauptplatine über eine abgeschirmte Leitung verbunden ist.
